# EUROPEAN PATENT APPLICATION

(11) **EP 3 670 729 A1**
(43) Date of publication of application: **24.06.2020**
(21) Application number: 19215866.5
(22) Date of filing: 13.12.2019
(51) Int. Cl.: D06F 33/43, D06F 33/69, D06F 34/14, D06F 35/00, D06F 58/45

(54) **LAUNDRY TREATMENT DEVICE AND METHOD OF OPERATING THE SAME**

(30) Priority: 19.12.2018 WO PCT/KR2018/016218
(71) Applicant: LG Electronics Inc., SEOUL, 07336 (KR)
(72) Inventor: JEONG, Hangil, 06772 Seoul (KR); KIM, Jaehong, 06772 Seoul (KR); KIM, Hyoeun, 06772 Seoul (KR); LEE, Taeho, 06772 Seoul (KR); HAN, Jongwoo, 06772 Seoul (KR)
(74) Representative: Katérle, Axel

(57) **Abstract**

A laundry treatment device (300) includes a wireless communication unit, at least one sensor, and a processor (180) configured to apply a learning model learned through a supervised learning algorithm to sensing information including a sensing value collected from the at least one sensor and a measurement time of the sensing value, to acquire microorganism information including a type of microorganism and a proliferation rate of the microorganism, to acquire laundry guide information based on the acquired microorganism information, and to transmit the acquired laundry guide information to a terminal through the wireless communication unit.

## Description

### FIELD

The present invention relates to a laundry treatment device, and, more particularly, to a laundry treatment device capable of determining a contamination state of the laundry treatment device using artificial intelligence based on sensing data.

### BACKGROUND

In modern times, laundry treatment devices are essential appliances in every home.

In recent years, laundry treatment devices having a sterilization function has been developed in view of consumer's growing awareness of sterilization.

Such laundry treatment devices having the sterilization function perform tub cleaning, operate a dehumidification mode or use ultraviolet rays, for sterilization.

However, a conventional laundry treatment device detected mold or bacteria and automatically performed tub cleaning, but did not take measures according to the type of mold or bacteria.

Since the laundry treatment device performed only across-the-board tub cleaning, tub cleaning suitable for the type of detected microorganism or a proliferation situation was not performed and thus cleaning may not be properly performed.

### SUMMARY

An object of the present invention is to take measures according to a contamination state of a laundry treatment device, by differentiating washing operation according to the type and proliferation rate of microorganism detected in the laundry treatment device.

A laundry treatment device according to an embodiment of the present invention may predict the type and proliferation rate of microorganism based on a sensing value of one or more sensors attached to the laundry treatment device and provide laundry guide information based on the predicted information.

### BRIEF DESCRIPTION OF THE DRAWINGS

FIG. 1 is a block diagram illustrating a learning apparatus of an artificial neural network.
FIG. 2 is a block diagram illustrating a terminal according to an embodiment of the present invention.
FIG. 3 is a block diagram showing the configuration of a laundry treatment device according to an embodiment of the present invention.
FIG. 4 is a flowchart illustrating a method of operating a laundry treatment device according to an embodiment of the present invention.
FIG. 5 is a view illustrating an example of sensing information collected by a sensing unit according to an embodiment of the present invention.
FIG. 6 is a view illustrating a method of acquiring the type of microorganism using a linear regression algorithm according to an embodiment of the present invention.
FIG. 7 is a view illustrating a method of acquiring different types of microorganism using a linear regression algorithm according to an embodiment of the present invention.
FIGS. 8 to 10 are views illustrating an example in which a terminal capable of communicating with a laundry treatment device displays microorganism information and laundry guide information according to an embodiment of the present invention.
FIG. 11 is a ladder diagram illustrating a method of operating a washing system according to an embodiment of the present invention.

### DETAILED DESCRIPTION OF THE EMBODIMENTS

Exemplary embodiments of the present invention will be described below in detail with reference to the accompanying drawings in which the same reference numbers are used throughout this specification to refer to the same or like parts and a repeated description thereof will be omitted. The suffixes "module" and "unit" of elements herein are used for convenience of description and thus can be used interchangeably and do not have any distinguishable meanings or functions. In describing the present invention, a detailed description of known functions and configurations will be omitted when it may obscure the subject matter of the present invention. The accompanying drawings are used to help easily understood the technical idea of the present invention and it should be understood that the idea of the present invention is not limited by the accompanying drawings. The idea of the present invention should be construed to extend to any alterations, equivalents and substitutions besides the accompanying drawings.

It will be understood that, although the terms first, second, etc. may be used herein to describe various elements of the present invention, these terms are only used to distinguish one element from another element and essential, order, or sequence of corresponding elements are not limited by these terms.

It will be understood that when one element is referred to as being "connected to" or "coupled to" another element, one element may be "connected to" or "coupled to", another element via a further element although one element may be directly connected to or directly accessed to another element.

Artificial intelligence (AI) is a field of computer engineering and information technology involving studying how computers can think, learn and self-develop in ways similar to human intelligence, and means that computers can emulate intelligent actions of humans.

In addition, artificial intelligence does not exist by itself but is directly or indirectly associated with the other fields of computer science. In particular, many attempts have been made to introduce elements of artificial intelligence into various fields of information technology.

Machine learning is a field of artificial intelligence, which gives a computer the ability to learn without explicit programming.

Specifically, machine learning refers to technology for studying and building a system for performing learning based on empirical data, performing prediction and improving performance thereof and an algorithm therefor. Machine learning algorithms do not perform strictly defined static program commands, but rather builds a specific model to make a prediction or decision based on input data.

The term machine learning may be used interchangeably with the term machine learning.

Many machine learning algorithms have been developed based on how to classify data in machine learning. Representative examples thereof include a decision tree, a Bayesian network, a support vector machine (SVM) and an artificial neural network.

The decision tree refers to an analysis method of performing classification and prediction by plotting decision rules in a tree structure.

The Bayesian network is a model for representing conditional independence between multiple variables in a graph structure. The Bayesian network is suitable for data mining through unsupervised learning.

The SVM is a model of supervised learning for pattern recognition and data analysis and is mainly used for classification and regression analysis.

The artificial neural network (ANN) is a model of a connection relationship between neurons and the operation principle of biological neurons and is an information processing system in which a plurality of neurons such as nodes or processing elements are connected in the form of layers.

The artificial neural network (ANN) is a model used for machine learning and is a statistical learning algorithm inspired by biological neural networks (especially, the brain of the central nervous system of the animal) in machine learning and cognitive science.

Specifically, the ANN may mean a model having a problem solution ability, by changing the strength of connection of the synapses through learning at artificial neurons (nodes) forming a network by connecting synapses.

The term artificial neural network (ANN) may be used interchangeably with the term neural network.

The ANN may include a plurality of layers and each layer may include a plurality of neurons. In addition, the ANN may include synapses connecting neurons.

The ANN may be generally defined by the following three factors: (1) a connection pattern between neurons of different layers, (2) a learning process of updating the weight of a connection, and (3) an activation function for generating an output value by a weighted sum of input received from a previous layer.

The ANN may include various network models such as a deep neural network (DNN), a recurrent neural network (RNN), a bidirectional recurrent deep neural network (BRDNN), a multilayer perceptron (MLP), and a convolutional neural network (CNN), without being limited thereto.

In this specification, the term layer may be used interchangeably with the term layer.

The ANN may be classified into single-layer neural networks and multilayer neural networks according to the number of layers.

A general single-layer neural network includes an input layer and an output layer.

In addition, a general multilayer neural network includes an input layer, a hidden layer and an output layer.

The input layer receives external data, and the number of neurons of the input layer is equal to the number of input variables. The hidden layer is located between the input layer and the output layer. The hidden layer receives a signal from the input layer, and extracts and transmits characteristics to the output layer. The output layer receives a signal from the hidden layer and outputs the signal to the outside.

The input signals of neurons are multiplied by respective strengths of connection having values between 0 and 1 and then are summed. If this sum is greater than a threshold value of the neuron, the neuron is activated and an output value is obtained through an activation function.

Meanwhile, a deep neural network (DNN) including a plurality of hidden layers between an input layer and an output layer may be a representative artificial neural network for implementing deep learning which is machine learning technology.

Meanwhile, the term deep learning may be used interchangeably with the term deep learning.

The ANN may be trained using training data. Here, training may mean a process of determining parameters of the ANN using training data for the purpose of classifying, regressing or clustering input data. The representative examples of the parameters of the ANN include a weight applied to a synapse and a bias applied to a neuron.

The ANN trained using the training data may classify or cluster input data according to the pattern of the input data.

Meanwhile, the ANN trained using the training data may be referred to as a trained model in this specification.

Next, a learning method of the ANN will be described.

The learning method of the ANN may be roughly classified into supervised learning, unsupervised learning, semi-supervised learning, and reinforcement learning.

The supervised learning is a method of deriving one function from training data.

Among the derived functions, outputting consecutive values may be referred to as regression and predicting and outputting the class of an input vector may be referred to as classification.

In the supervised learning, the ANN is trained in a state in which training data is labeled.

Here, the label may mean a correct answer (or a result value) inferred by an ANN when training data is input to the ANN.

In this specification, the correct answer (or the result value) inferred by the ANN when training data is input is referred to as a label or labeling data.

In this specification, labeling training data for training the ANN is referred to as labeling training data with labeling data.

In this case, training data and a label corresponding to the training data configure one training set, and the training set may be input to the ANN.

Meanwhile, the training data represents a plurality of features and labeling the training data may mean labeling the feature represented by the training data. In this case, the training data may represent the feature of an input object in the form of a vector.

The ANN may derive a function of an association between training data and labeling data using the training data and the labeling data. In addition, the ANN may determine (optimize) the parameter of the ANN through evaluation of the derived function.

The unsupervised learning is a kind of machine learning and training data is not labeled.

Specifically, the unsupervised learning may be a method of training the ANN to find and classify a pattern in the training data itself rather than the association between the training data and the label corresponding to the training data.

Examples of the unsupervised learning may include clustering and independent component analysis.

In this specification, the term clustering may be used interchangeably with the term clustering.

Examples of an ANN using unsupervised learning may include a generative adversarial network (GAN) and an autoencoder (AE).

The GAN refers to a machine learning method of improving performance through competition between two different artificial intelligence models, that is, a generator and a discriminator.

In this case, the generator is a model for generating new data and may generate new data based on original data.

In addition, the discriminator is a model for discriminating the pattern of data and may discriminate authenticity of the new data generated by the generator based on the original data.

The generator may receive and learn data which does not deceive the discriminator, and the discriminator may receive and learn deceiving data from the generator. Accordingly, the generator may evolve to maximally deceive the discriminator and to distinguish between the original data of the discriminator and the data generated by the generator.

The autoencoder (AE) is a neural network which aims to reproduce input itself as output.

The AE includes an input layer, a hidden layer and an output layer. Input data is input to the hidden layer through the input layer.

In this case, since the number of nodes of the hidden layer is less than the number of nodes of the input layer, the dimension of data is reduced and thus compression or encoding is performed.

Meanwhile, the AE controls the strength of connection of the neuron through learning, such that input data is represented by hidden-layer data. In the hidden layer, information is represented by a smaller number of neurons than the input layer, and reproducing input data as output may mean that the hidden layer finds a hidden pattern from the input data and expresses the hidden pattern.

The semi-supervised learning is a kind of machine learning and may refer to a learning method of using both labeled training data and unlabeled training data.

As one of the semi-supervised learning technique, there is a technique for inferring the label of unlabeled training data and then performing learning using the inferred label. This technique is useful when labeling cost is high.

Reinforcement learning is a theory that an agent can find the best way through experience without data when an environment in which the agent may decide what action is taken every moment is given.

Reinforcement learning may be performed by a Markov decision process (MDP).

The Markov Decision Process (MDP) will be briefly described. First, an environment including information necessary for the agent to take a next action is given. Second, what action is taken by the agent in that environment is defined. Third, a reward given to the agent when the agent successfully takes a certain action and a penalty given to the agent when the agent fails to take a certain action are defined. Fourth, experience is repeated until a future reward reaches a maximum point, thereby deriving an optimal action policy.

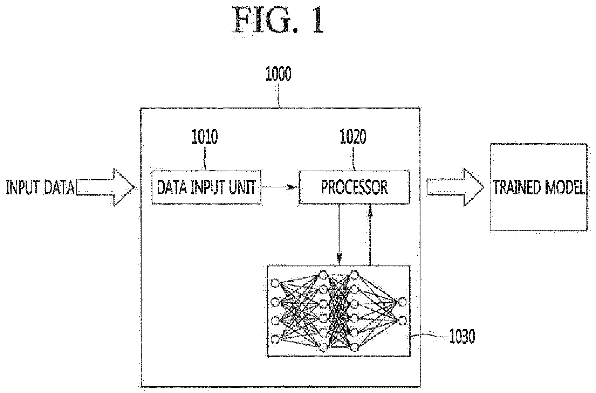

FIG. 1 is a block diagram illustrating a learning apparatus of an artificial neural network.

The learning apparatus 1000 of the artificial neural network may include a data input unit 1010, a processor 1020 and an artificial neural network 1030.

The data input unit 1010 may receive input data. In this case, the data input unit 1010 may receive training data or unprocessed data.

When the data input unit 1010 receives unprocessed data, the processor 1020 may preprocess the received data and generate training data capable of being input to the artificial neural network 1030.

The artificial neural network 1030 may be implemented in hardware, software or a combination thereof. If a portion or whole of the artificial neural network 1030 is implemented in software, one or more commands configuring the artificial neural network 1030 may be stored in a memory (not shown) included in the learning apparatus 1000 of the artificial neural network.

The processor 1020 may input training data or a training set to the artificial neural network 1030 to train the artificial neural network 1030.

Specifically, the processor 1020 may repeatedly train the artificial neural network (ANN) using various learning methods, thereby determining (optimizing) parameters of the artificial neural network (ANN).

The artificial neural network having the parameters determined by learning using the training data may be referred to as a trained model.

Meanwhile, the trained model may be used to infer a result value for new input data instead of the training data.

Meanwhile, the trained model may infer the result value in a state of being installed in the learning apparatus 1000 of the artificial neural network and may be transmitted to and installed in another device.

When the trained model is transmitted to another device, the learning apparatus 1000 of the artificial neural network may include a communication unit (not shown) for communication with another device.

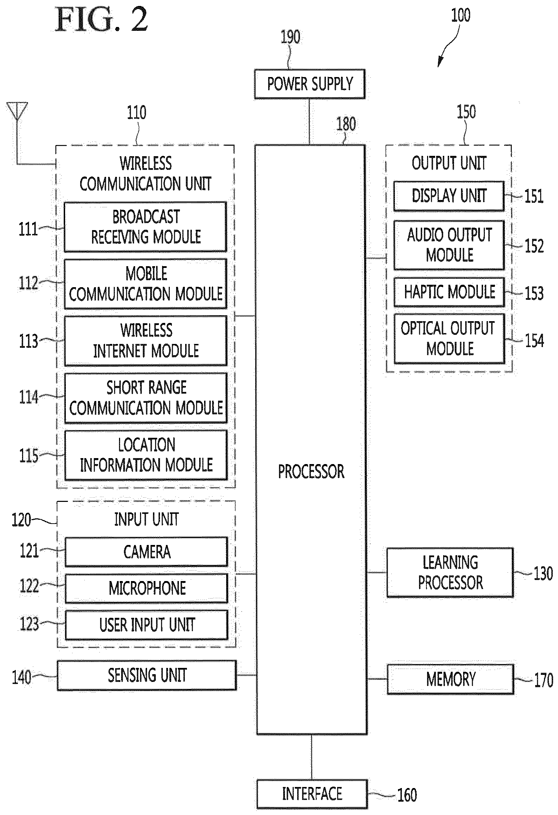

FIG. 2 is a block diagram illustrating a terminal according to an embodiment of the present invention.

The terminal described in this specification may include cellular phones, smart phones, laptop computers, digital broadcast terminals, personal digital assistants (PDAs), portable multimedia players (PMPs), navigators, portable computers (PCs), slate PCs, tablet PCs, ultra books, wearable devices (for example, smart watches, smart glasses, head mounted displays (HMDs)), and the like.

However, the terminal 100 according to the embodiment is applicable to stationary terminals such as smart TVs, desktop computers or digital signages.

In addition, the terminal 100 according to the embodiment of the present invention is applicable to stationary or mobile robots.

In addition, the terminal 100 according to the embodiment of the present invention may perform the function of a voice agent. The voice agent may be a program for recognizing the voice of a user and audibly outputting a response suitable to the recognized voice of the user.

The terminal 100 may include a wireless communication unit 110, an input unit 120, a learning processor 130, a sensing unit 140, an output unit 150, an interface 160, a memory 170, a processor 180 and a power supply 190.

The trained model may be installed in the terminal 100.

Meanwhile, the trained model may be implemented in hardware, software or a combination thereof. If a portion or whole of the trained model is implemented in software, one or more commands configuring the trained model may be stored in the memory 170.

The wireless communication unit 110 may include at least one of a broadcast reception module 111, a mobile communication module 112, a wireless Internet module 113, a short-range communication module 114 and a location information module 115.

The broadcast reception module 111 receives broadcast signals and/or broadcast associated information from an external broadcast management server through a broadcast channel.

The mobile communication module 112 can transmit and/or receive wireless signals to and from at least one of a base station, an external terminal, a server, and the like over a mobile communication network established according to technical standards or communication methods for mobile communication (for example, Global System for Mobile Communication (GSM), Code Division Multi Access (CDMA), CDMA2000 (Code Division Multi Access 2000), EV-DO (Enhanced Voice-Data Optimized or Enhanced Voice-Data Only), Wideband CDMA (WCDMA), High Speed Downlink Packet access (HSDPA), HSUPA (High Speed Uplink Packet Access), Long Term Evolution (LTE), LTE-A (Long Term Evolution-Advanced), and the like).

The wireless Internet module 113 is configured to facilitate wireless Internet access. This module may be installed inside or outside the mobile terminal 100. The wireless Internet module 113 may transmit and/or receive wireless signals via communication networks according to wireless Internet technologies.

Examples of such wireless Internet access include Wireless LAN (WLAN), Wireless Fidelity (Wi-Fi), Wi-Fi Direct, Digital Living Network Alliance (DLNA), Wireless Broadband (WiBro), Worldwide Interoperability for Microwave Access (WiMAX), High Speed Downlink Packet Access (HSDPA), HSUPA (High Speed Uplink Packet Access), Long Term Evolution (LTE), LTE-A (Long Term Evolution-Advanced), and the like.

The short-range communication module 114 is configured to facilitate short-range communication and to support short-range communication using at least one of Bluetooth™, Radio Frequency IDentification (RFID), Infrared Data Association (IrDA), Ultra-WideBand (UWB), ZigBee, Near Field Communication (NFC), Wireless-Fidelity (Wi-Fi), Wi-Fi Direct, Wireless USB (Wireless Universal Serial Bus), and the like.

The location information module 115 is generally configured to acquire the position (or the current position) of the terminal. Representative examples thereof include a Global Position System (GPS) module or a Wi-Fi module. As one example, when the mobile terminal uses a GPS module, the position of the terminal may be acquired using a signal sent from a GPS satellite.

The input unit 120 may include a camera 121 for receiving a video signal, a microphone 122 for receiving an audio signal, and a user input unit 123 for receiving information from a user.

Voice data or image data collected by the input unit 120 may be analyzed and processed as a control command of the user.

The input unit 120 may receive video information (or signal), audio information (or signal), data or user input information. For reception of video information, the mobile terminal 100 may include one or a plurality of cameras 121.

The camera 121 may process image frames of still images or moving images obtained by image sensors in a video call more or an image capture mode. The processed image frames can be displayed on the display 151 or stored in memory 170.

The microphone 122 processes an external acoustic signal into electrical audio data. The processed audio data may be variously used according to function (application program) executed in the mobile terminal 100. If desired, the microphone 122 may include various noise removal algorithms to remove noise generated in the process of receiving the external acoustic signal.

The user input unit 123 receives information from a user.

The processor 180 may control operation of the terminal 100 in correspondence with the input information.

The user input unit 123 may include one or more of a mechanical input element (for example, a mechanical key, a button located on a front and/or rear surface or a side surface of the mobile terminal 100, a dome switch, a jog wheel, a jog switch, and the like) or a touch input element. As one example, the touch input element may be a virtual key, a soft key or a visual key, which is displayed on a touchscreen through software processing, or a touch key located at a location other than the touchscreen.

The learning processor 130 may be configured to receive, classify, store and output information to be used for data mining, data analysis, intelligent decision, mechanical learning algorithms and techniques.

The learning processor 130 may include one or more memory units configured to store data received, detected, sensed, generated or output in a predetermined manner or another manner by the terminal or received, detected, sensed, generated or output in a predetermined manner or another manner by another component, device, terminal or device for communicating with the terminal.

The learning processor 130 may include a memory integrated with or implemented in the terminal. In some embodiment, the learning processor 130 may be implemented using the memory 170.

Selectively or additionally, the learning processor 130 may be implemented using a memory related to the terminal, such as an external memory directly coupled to the terminal or a memory maintained in a server communicating with the terminal.

In another embodiment, the learning processor 130 may be implemented using a memory maintained in a cloud computing environment or another remote memory accessible by the terminal through the same communication scheme as a network.

The learning processor 130 may be configured to store data in one or more databases in order to identify, index, categorize, manipulate, store, retrieve and output data to be used for supervised or unsupervised learning, data mining, predictive analysis or other machines.

Information stored in the learning processor 130 may be used by one or more other controllers of the terminal or the processor 180 using any one of different types data analysis algorithms and machine learning algorithms.

Examples of such algorithms include k-nearest neighbor systems, fuzzy logic (e.g., possibility theory), neural networks, Boltzmann machines, vector quantization, pulse neural networks, support vector machines, maximum margin classifiers, hill climbing, inductive logic system Bayesian networks, Petri Nets (e.g., finite state machines, Mealy machines or Moore finite state machines), classifier trees (e.g., perceptron trees, support vector trees, , Marcov trees, decision tree forests, random forests), betting models and systems, artificial fusion, sensor fusion, image fusion, reinforcement learning, augmented reality, pattern recognition, and automated planning.

The processor 180 may make a decision using data analysis and machine learning algorithms and determine or predict at least one executable operation of the terminal based on the generated information. To this end, the processor 180 may request, retrieve, receive or use the data of the processor 130 and control the terminal to execute preferable operation or predicted operation of at least executable operation.

The processor 180 may perform various functions for implementing intelligent emulation (that is, a knowledge based system, an inference system and a knowledge acquisition system). This is applicable to various types of systems (e.g., a fussy logic system) including an adaptive system, a machine learning system, an artificial neural system, etc.

The processor 180 may include a sub module enabling operation involving speech and natural language speech processing, such as an I/O processing module, an environmental condition module, speech-to-text (STT) processing module, a natural language processing module, a workflow processing module and a service processing module.

Each of such sub modules may have an access to one or more systems or data and models at the terminal or a subset or superset thereof. In addition, each of the sub modules may provide various functions including vocabulary index, user data, a workflow model, a service model and an automatic speech recognition (ASR) system.

In another embodiment, the other aspects of the processor 180 or the terminal may be implemented through the above-described sub modules, systems or data and models.

In some embodiments, based on the data of the learning processor 130, the processor 180 may be configured to detect and sense requirements based on the context condition or user's intention expressed in user input or natural language input.

The processor 180 may actively derive and acquire information necessary to fully determine the requirements based on the context condition or user's intention. For example, the processor 180 may actively derive information necessary to determine the requirements, by analyzing historical data including historical input and output, pattern matching, unambiguous words, and input intention, etc.

The processor 180 may determine a task flow for executing a function for responding to the requirements based on the context condition or the user's intention.

The processor 180 may be configured to collect, sense, extract, detect and/or receive signals or data used for data analysis and machine learning operations through one or more sensing components at the terminal, in order to collect information for processing and storage from the learning processor 130.

Information collection may include sensing information through a sensor, extracting information stored in the memory 170, or receiving information from another terminal, an entity or an external storage device through a communication unit.

The processor 180 may collect and store usage history information from the terminal.

The processor 180 may determine the best match for executing a specific function using the stored usage history information and predictive modeling.

The processor 180 may receive or sense surrounding information or other information through the sensing unit 140.

The processor 180 may receive broadcast signals and/or broadcast related information, wireless signals or wireless data through the wireless communication unit 110.

The processor 180 may receive information (or signals corresponding thereto), audio signal (or signals corresponding thereto), data or user input information from the input unit 120.

The processor 180 may collect information in real time, process or classify the information (e.g., a knowledge graph, a command policy, a personalization database, a dialog engine, etc.), and store the processed information in the memory 170 or the learning processor 130.

When the operation of the terminal is determined based on data analysis and machine learning algorithms and techniques, the processor 180 may control the components of the terminal in order to execute the determined operation. The processor 180 may control the terminal according to a control command and perform the determined operation.

When the specific operation is performed, the processor 180 may analyze historical information indicating execution of the specific operation through data analysis and machine learning algorithms and techniques and update previously learned information based on the analyzed information.

Accordingly, the processor 180 may improve accuracy of future performance of data analysis and machine learning algorithms and techniques based on the updated information, along with the learning processor 130.

The sensing unit 140 may include one or more sensors configured to sense internal information of the terminal, the surrounding environment of the terminal, user information, and the like.

For example, the sensing unit 140 may include at least one of a proximity sensor 141, an illumination sensor 142, a touch sensor, an acceleration sensor, a magnetic sensor, a G-sensor, a gyroscope sensor, a motion sensor, an RGB sensor, an infrared (IR) sensor, a fingerprint (finger scan) sensor, an ultrasonic sensor, an optical sensor (for example, a camera 121), a microphone 122, a battery gauge, an environment sensor (for example, a barometer, a hygrometer, a thermometer, a radiation detection sensor, a thermal sensor, and a gas sensor), and a chemical sensor (for example, an electronic nose, a health care sensor, a biometric sensor, and the like). The terminal disclosed in this specification may be configured to combine and utilize information obtained from at least two sensors of such sensors.

The output unit 150 is typically configured to output various types of information, such as audio, video, tactile output, and the like. The output unit 150 may include a display 151, an audio output module 152, a haptic module 153, and an optical output module 154.

The display 151 is generally configured to display (output) information processed in the mobile terminal 100. For example, the display 151 may display execution screen information of an application program executed by the mobile terminal 100 or user interface (UI) and graphical user interface (GUI) information according to the executed screen information.

The display 151 may have an inter-layered structure or an integrated structure with a touch sensor in order to realize a touchscreen. The touchscreen may provide an output interface between the mobile terminal 100 and a user, as well as function as the user input unit 123 which provides an input interface between the mobile terminal 100 and the user.

The audio output module 152 is generally configured to output audio data received from the wireless communication unit 110 or stored in the memory 170 in a call signal reception mode, a call mode, a record mode, a voice recognition mode, a broadcast reception mode, and the like.

The audio output module 152 may also include a receiver, a speaker, a buzzer, or the like.

A haptic module 153 can be configured to generate various tactile effects that a user feels. A typical example of a tactile effect generated by the haptic module 153 is vibration.

An optical output module 154 can output a signal for indicating event generation using light of a light source of the mobile terminal 100. Examples of events generated in the mobile terminal 100 may include message reception, call signal reception, a missed call, an alarm, a schedule notice, email reception, information reception through an application, and the like.

The interface 160 serves as an interface with external devices to be connected with the mobile terminal 100. The interface 160 may include wired or wireless headset ports, external power supply ports, wired or wireless data ports, memory card ports, ports for connecting a device having an identification module, audio input/output (I/O) ports, video I/O ports, earphone ports, or the like. The terminal 100 may perform appropriate control related to the connected external device in correspondence with connection of the external device to the interface 160.

The identification module may be a chip that stores a variety of information for granting use authority of the mobile terminal 100 and may include a user identity module (UIM), a subscriber identity module (SIM), a universal subscriber identity module (USIM), and the like. In addition, the device having the identification module (also referred to herein as an "identifying device") may take the form of a smart card. Accordingly, the identifying device can be connected with the terminal 100 via the first interface 160.

The memory 170 stores data supporting various functions of the terminal 100.

The memory 170 may store a plurality of application programs or applications executed in the terminal 100, data and commands for operation of the terminal 100, and data for operation of the learning processor 130 (e.g., at least one piece of algorithm information for machine learning).

The processor 180 generally controls overall operation of the terminal 100, in addition to operation related to the application program. The processor 180 may process signals, data, information, etc. input or output through the above-described components or execute the application program stored in the memory 170, thereby processing or providing appropriate information or functions to the user.

In addition, the processor 180 may control at least some of the components described with reference to FIG. 1 in order to execute the application program stored in the memory 170. Further, the processor 180 may operate a combination of at least two of the components included in the terminal, in order to execute the application program.

The power supply 190 receives external power or internal power and supplies the appropriate power required to operate respective components included in the mobile terminal 100, under control of the controller 180. The power supply 190 may include a battery, which is typically rechargeable or be detachably coupled to the terminal body for charging.

Meanwhile, as described above, the processor 180 controls operation related to the application program and overall operation of the terminal 100. For example, the processor 180 may execute or release a lock function for limiting input of a control command of the user to applications when the state of the terminal satisfies a set condition.

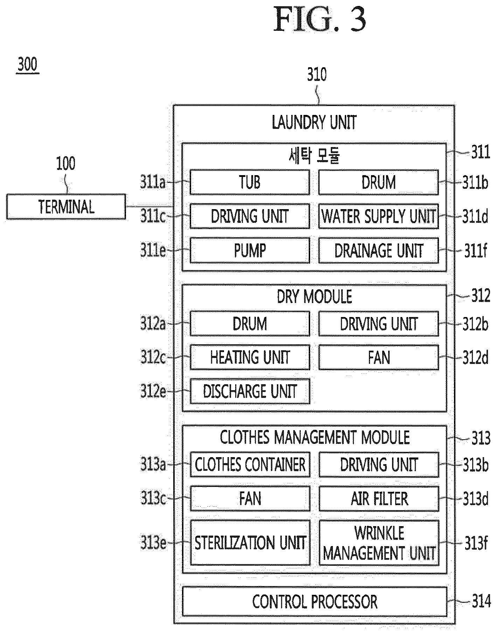

FIG. 3 is a block diagram showing the configuration of a laundry treatment device according to an embodiment of the present invention.

Referring to FIG. 3, the laundry treatment device 300 may include the terminal 100 shown in FIG. 2 and a laundry unit 310.

The terminal 100 may be modularized and configured as an internal component of the laundry treatment device 300.

The laundry treatment device 300 may include the internal components of the terminal 100 shown in FIG. 2 and the laundry unit 310 as parallel components.

The laundry unit 310 may include at least one of a washing module 311 for performing a function related to washing, a dry module 312 for performing a function related to dry or a clothes management module 313 for performing a function related to clothes management.

The washing module 311 may perform functions related to immersion, washing, rinsing and dehydration.

The dry module 312 may perform a function for drying laundry using various methods. Typically, laundry may be dried using wind (hot air or cold air).

The clothes management module 313 may perform functions a variety of clothes management such as clothes hanging, dry cleaning, dust removal, sterilization, wrinkle removal and ironing.

The processor 180 or a control processor 314 provided in the laundry unit 310 may control components included in the washing module 311, the dry module 312 or the clothes management module 313 of the laundry unit 310 to provide various washing functions.

The input unit 120 and the sensing unit 140 may collect data related to interaction with a user related to use and control of the laundry unit 310. For example, the input unit 120 and the sensing unit 140 may collect course selection information and control information through voice or interaction.

The output unit 150 may output information related to use and control of the laundry unit 310. For example, the output unit 150 may output course information, use record, control information, etc. corresponding to washing, drying and clothes management.

The memory 170 may store information related to use and control of the laundry unit 310. For example, the memory 170 may store course information, use record, control information, etc. corresponding to washing, drying and clothes management.

Specifically, the washing module 311 may include a tub 311a in which wash water is stored, a drum 311b rotatably mounted in the tub to have laundry put thereinto, a driving unit 311c for rotating the drum, a water supply unit 311d for supplying wash water, a pump 311e for circulating or discharging wash water, a drainage unit 311f for discharging wash water, etc.

The drum 311b in which laundry is received may be rotatably provided in the tub 311a. The drum 311b receives laundry, has an inlet located at a front surface or an upper surface thereof such that laundry is put therethrough, and rotates around a substantially horizontal or vertical rotation center line. A plurality of through-holes may be formed in the drum 311b such that water in the tub 311a flows into the drum 311b.

The terms "horizontal" or "vertical" used herein are not used in the mathematically strict sense. That is, as in the embodiment, a rotation center line inclined from the horizontal or vertical direction by a predetermined angle is close to the horizontal or vertical direction and thus may be said to be substantially horizontal or vertical.

The water supply unit 311d may include a water supply valve, a water supply pipe and a water supply hose, etc.

When water is supplied, wash water passing through the water supply valve and the water supply pipe may be mixed with a detergent in a detergent dispenser and supplied to the tub 311a through the water supply hose.

Meanwhile, a direct water supply pipe may be connected to the water supply valve such that wash water is directly supplied into the tub 311a through the direct water supply pipe without being mixed with the detergent.

The pump 311e performs functions of a drainage pump 311e for discharging wash water to the outside and a circulation pump 311e for circulating wash water. Alternatively, the drainage pump 311e and the circulation pump 311e may be separately provided.

The pump 311e may be connected to a drainage pipe provided in the drainage unit 31 If to discharge wash water to the outside through the drainage pipe. In addition, the pump 311e may be connected to a circulated water supply pipe to spray wash water stored in the tub 311a into the drum 311b through the circulated water supply pipe, thereby circulating wash water.

One or more protrusions protruding toward the inside of the drum 311b may be provided on the inner surface of the drum 311b.

The protrusions may be a lifter disposed on the inner surface of the drum 311b or an integrally formed embossing. If the lifter or the embossing is formed on the inner surface of the drum 311b, the laundry may be repeatedly lifted up or down by the lifter when the drum 311b rotates.

The driving unit 311c may rotate the drum 311b, and the driving shaft rotated by the driving unit 311 c may be coupled to the drum 311b through the rear surface of the tub 311a.

The driving unit 311 c may include a motor capable of speed control.

At this time, the motor may be an inverter direct drive motor.

The control processor 314 may receive the output value (e.g., output current) of the motor of the driving unit 311c and control the rotation number (or the rotation speed) of the motor of the driving unit 311c to follow a predetermined target rotation number (or a target rotation speed) based on the output value. In addition, the control processor 314 may control driving of the motor of the driving unit 311c according to a driving pattern.

In addition, the dry module 312 may include a drum 312b in which laundry is put, a driving unit 312b for rotating the drum, a heating unit 312c for heating air, a fan 312d for circulating inside air, a discharge unit 312e for discharging inside air, etc.

The drum 312a is a space in which a material to be dried is dried, and is rotatably provided in a main body. In addition, the drum may be provided with one or more lifters for lifting up or down the material to be dried.

The drum 312a is connected to an intake port (not shown) and air may be introduced to the inside by the fan 312d.

The driving unit 312b may rotate the drum 312a, and the driving shaft rotated by the driving unit 312b may be coupled to the drum 312a.

The driving unit 312b may include a motor capable of speed control.

At this time, the motor may be an inverter direct drive motor.

The control processor 314 may receive the output value (e.g., output current) of the motor of the driving unit 312b and control the rotation number (or the rotation speed) of the motor of the driving unit 312b to follow a predetermined target rotation number (or a target rotation speed) based on the output value. In addition, the control processor 314 may control driving of the motor of the driving unit 312b according to a driving pattern.

The heating unit 312c may include a heating part for heating air inside the drum 312a or air introduced from the outside.

The heating part may heat air using various energy sources such as a gas type or an electric type. In the case of an electric type, a coil heater may be used.

The heating unit 312c may include a plurality of heating parts, and the heating parts may not be equal to each other, may use various energy sources, and may have different performances.

The fan 312d circulates air heated by the heating unit 312c or air in the drum 312a.

The discharge unit 312e serves to guide the air inside the drum 312a to the outside, and may include an exhaust duct, an air filter, etc.

In addition, the clothes management module 313 may include a clothes container 313a in which clothes are held, a driving unit 313b for moving a holder provided in the clothes container 313a, a fan 313c for circulating inside air, an air filter 313d, a sterilization unit 313e and a wrinkle management unit 313f.

The clothes container 313a is a space for accommodating clothes (laundry) to be managed or treated and may include a holder for holding clothes. For example, the clothes container may include a hanger or a hook for hanging the hanger or a three-dimensional structure such as a torso or a mannequin.

The clothes container 313a is connected to an intake port (not shown) and air may be introduced to the inside by the fan 313c.

The driving unit 313b may drive the holder to induce predetermined movement of clothes held by the holder.

For example, the driving unit 313b may operate such that the holder and clothes held by the holder vibrate according to a certain vibration pattern. As the held laundry vibrates, dust or foreign materials attached or adhered to clothes may be removed.

The driving unit 313b may include a motor capable of speed control.

At this time, the motor may be an inverter direct drive motor.

The control processor 314 may receive the output value (e.g., output current) of the motor of the driving unit 313b and control the rotation number (or the rotation speed) of the motor of the driving unit 313b to follow a predetermined target rotation number (or a target rotation speed) based on the output value. In addition, the control processor 314 may control driving of the motor of the driving unit 313b according to a driving pattern.

The fan 313c circulates air introduced from the outside of the clothes container 313a or air inside the clothes container 313a.

The fan 313c may be provided so as to hit the supplied air on the clothes held in the clothes container 313a or to control an air supply direction.

For example, the fan 313c may blow air onto the held clothes to separate dust attached or adhered to the clothes from the clothes or to remove the moisture of the clothes.

The air filter 313d filters out dust, etc. when the inside air of the clothes container 313a is circulated or when the inside air is discharged to the outside.

The sterilization unit 313e may include various sterilization devices for sterilizing the held clothes.

For example, the sterilization devices may include a sterilization device using ozone and a sterilization device using ultraviolet rays.

The wrinkle management unit 313f may reduce or eliminate wrinkles of the held clothes and include a steam supply unit, an iron and an ironing board.

The steam supply unit may heat supplied water to make steam and naturally supply the steam to the clothes container 313a or directly spray the steam to clothes.

The iron and the ironing board are provided inside the clothes container 313a, and operation thereof may be controlled according to ironing information determined in consideration of the shape, size and materials of clothes to be ironed.

At this time, ironing information may include the position/motion line of the iron and the ironing board, ironing temperature/time, etc.

The control processor 314 may control the driving unit 313b or a driving unit provided in the wrinkle management unit 313f to control motion of the iron and the ironing board, and control the iron and the ironing board according to ironing information.

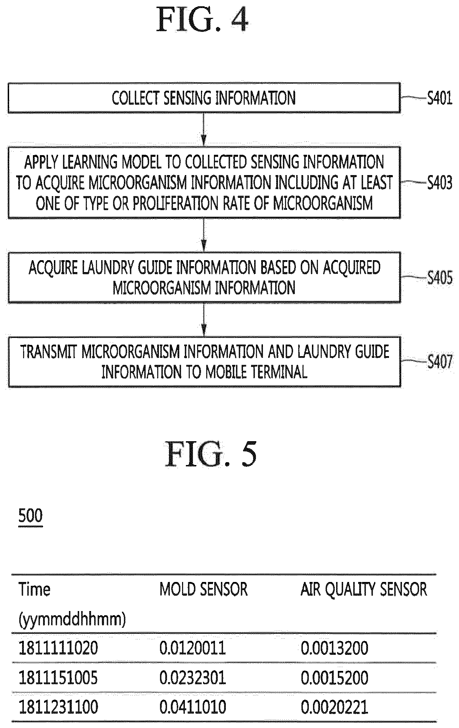

FIG. 4 is a flowchart illustrating a method of operating a laundry treatment device according to an embodiment of the present invention.

Referring to FIG. 4, the sensing unit 140 of the laundry treatment device 300 collects sensing information (S401).

In one embodiment, the sensing unit 140 may include at least one of a mold sensor (not shown), an air quality sensor (not shown) or a gas sensor.

In addition, one or more mold sensors, one or more air quality sensors or one or more gas sensors may be provided inside the laundry treatment device 300.

The sensing information may be collected in order to determine the type of microorganism or the proliferation rate of the microorganism such as mold or bacteria.

The sensing information may include a sensing value measured through the mold sensor, a sensing value measured through the air quality sensor and information on a time when each sensing value is measured.

This will be described with reference to FIG. 5.

FIG. 5 is a view illustrating an example of sensing information collected by a sensing unit according to an embodiment of the present invention.

Referring to FIG. 5, the sensing information 500 collected by the sensing unit 140 is shown.

For example, the sensing information 500 may include a first sensing value 0.0120011 measured through the mold sensor, a second sensing value 0.0013200 measured through the air quality sensor and a time 1811111020 (indicating November 11, 2018, 10:20) when the first sensing value and the second sensing value are measured.

The sensing unit 140 may periodically collect sensing information. Here, the period may be 1 minutes, but this is only an example.

FIG. 4 will be described again.

The processor 180 of the laundry treatment device 300 applies a learning model to the collected sensing information to acquire microorganism information including the type of the microorganism or the proliferation rate of the microorganism (S403).

In one embodiment, the learning model may be a model in which relationships between sensing information and the types of microorganism are learned.

In another embodiment, the learning model may be a model in which relationships between sensing information, the types of microorganism and the proliferation rate of the microorganism are learned.

The learning model may be obtained through a supervised learning method of learning data given a correct answer. More specifically, the learning model may be obtained through a linear regression algorithm which is a supervised learning method.

The linear regression algorithm refers to an algorithm for arbitrarily drawing a straight line of a linear function and predicting resultant data based on the straight line.

The processor 180 may receive the collected sensing information as input data and acquire one or more of the type of the microorganism or the proliferation rate of the microorganism matching the sensing information using the learning model.

In one embodiment, the learning model may be generated by the learning processor 130.

In another embodiment, the learning model may be received from an external server, for example, the learning apparatus 1000 of FIG. 1.

Meanwhile, the processor 180 may determine that new microorganism is detected, when the sensing information does not belong to the range of the sensing information included in the learning model.

In this case, the processor 180 may compare the range of the sensing values corresponding to the types of the microorganism with the collected sensing value and classify the microorganism corresponding to the sensing value to the type of the microorganism corresponding to the range of the sensing value closest to the collected sensing value.

A process of applying the learning model to the collected sensing information to acquire the microorganism information will be described in detail.

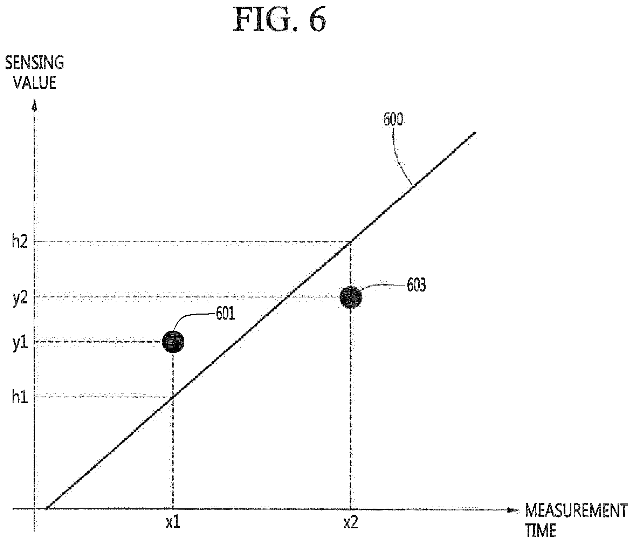

FIG. 6 is a view illustrating a method of acquiring the type of microorganism using a linear regression algorithm according to an embodiment of the present invention.

Referring to FIG. 6, a linear function 600 in which a relationship between a measurement time corresponding to mold A and a sensing value is learned is shown.

A learned linear function of mold B may have a slope and a y-intercept value different from those of the linear function 600 shown in FIG. 6.

The x-axis of the linear function 600 represents the measurement time of the sensing value and the y-axis represents the sensing value.

For example, a point having a first sensing value at a first time x1 is referred to as a first point 601 and a point having a second sensing value at a second time x2 is referred to as a second point 603.

The processor 180 may represent the collected first sensing information at a first point 601 and represent the collected second sensing information at a second point 603.

The processor 180 may determine that mold A has been detected, when a difference between y1 which is the y value of the first point 601 and h1 which is the y value of the linear function 600 is less than a reference value at the time x1 and a difference between y2 which is the y value of the second point 602 and h2 which is the y value of the linear function 600 is less than a reference value at the time x2.

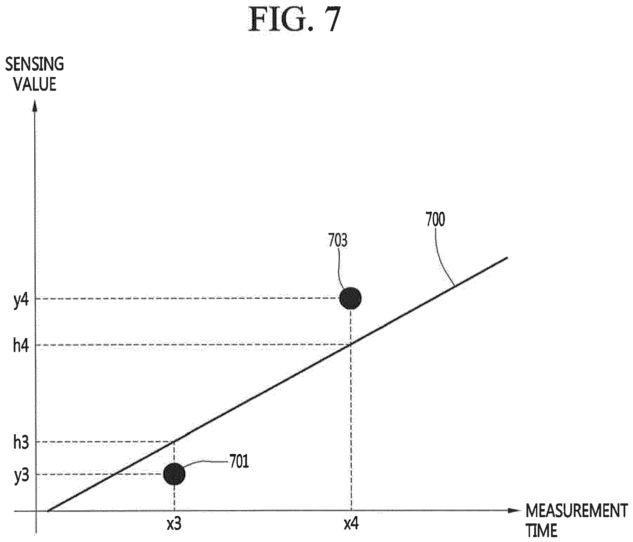

FIG. 7 is a view illustrating a method of acquiring different types of microorganism using a linear regression algorithm according to an embodiment of the present invention.

Referring to FIG. 7, a linear function 700 in which a relationship between a measurement time corresponding to mold B and a sensing value is learned is shown.

A learned linear function of mold B may have a slope and a y-intercept value different from those of the linear function 600 shown in FIG. 7.

The x-axis of the linear function 700 represents the measurement time of the sensing value and the y-axis represents the sensing value.

For example, a point having a third sensing value at a third time x3 is referred to as a third point 701 and a point having a fourth sensing value at a fourth time x4 is referred to as a fourth point 703.

The processor 180 may represent the collected third sensing information at a third point 701 and represent the collected fourth sensing information at a fourth point 703.

The processor 180 may determine that mold B has been detected, when a difference between y3 which is the y value of the third point 701 and h3 which is the y value of the linear function 700 is less than a reference value at the time x3 and a difference between y4 which is the y value of the fourth point 703 and h4 which is the y value of the linear function 700 is less than a reference value at the time x4.

FIG. 4 will be described again.

The processor 180 of the laundry treatment device 300 acquires laundry guide information based on the acquired microorganism information (S405).

In one embodiment, the processor 180 may acquire laundry guide information for guiding the user to perform specific washing operation in order to eliminate microorganism or to prevent proliferation of microorganism, based on microorganism information including at least one of the type of the microorganism or the proliferation rate of the microorganism.

The processor 180 may pre-store a table associating the laundry guide information with the type of the microorganism and the proliferation rate of the microorganism in the memory 170.

When the type of the microorganism and the proliferation rate of the microorganism are determined, the processor 180 may acquire the laundry guide information corresponding to the determined type and proliferation rate of the microorganism, using the table stored in the memory 170.

In another example, the laundry guide information may include the position information of a sensor for detecting the microorganism. To this end, the mold sensor or the air quality sensor of the sensing unit 140 may transmit identification information thereof to the processor 180 along with the sensing information.

The identification information may be included in the sensing information.

The identification information may include an identifier for identifying the sensor and the position information of the sensor indicating the position of the sensor in the laundry treatment device 300.

The processor 180 may include the identification information of the sensor for providing the sensing information, which is the basis for the microorganism information, in the microorganism information, when the microorganism information is acquired.

Thereafter, the processor 180 may determine the position of the sensor through the identification information of the sensor included in the microorganism information and generate the laundry guide information using the determined position of the sensor.

For example, when the sensor is disposed in the tub through the position information of the sensor, the laundry guide information may further include the position information of the microorganism indicating that the microorganism has been detected in the tub.

The processor 180 of the laundry treatment device 300 transmits the acquired microorganism and laundry guide information to the terminal 100 through the wireless communication unit 110 (S407).

The terminal 100 may display the microorganism information and the laundry guide information through the display 151.

In another example, if the display is provided in the laundry treatment device 300, the processor 180 may display the microorganism information and the laundry guide information on the display.

In another example, if a speaker is provided in the laundry treatment device 300, the processor 180 may audibly output the microorganism information and the laundry guide information through the speaker.

In another example, the processor 180 may control operation of the washing unit 310 to perform washing operation according to the laundry guide information.

For example, when the laundry guide information includes tub cleaning, the processor 180 may control operation of the washing module 311 to automatically perform tub cleaning.

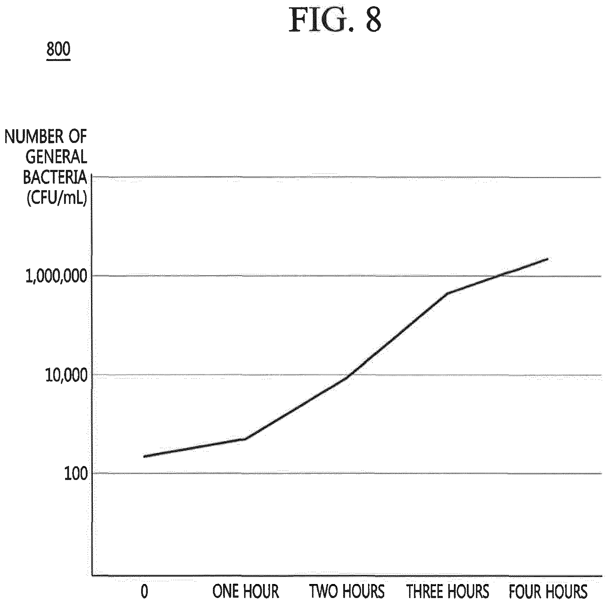

FIGS. 8 to 10 are views illustrating an example in which a terminal capable of communicating with a laundry treatment device displays microorganism information and laundry guide information according to an embodiment of the present invention.

Referring to FIG. 8, a graph 800 representing the proliferation rate of mold A is shown.

The user may see the predicted proliferation rate of mold A and obtain a motivation for dehumidification or cleaning of the laundry treatment device 300.

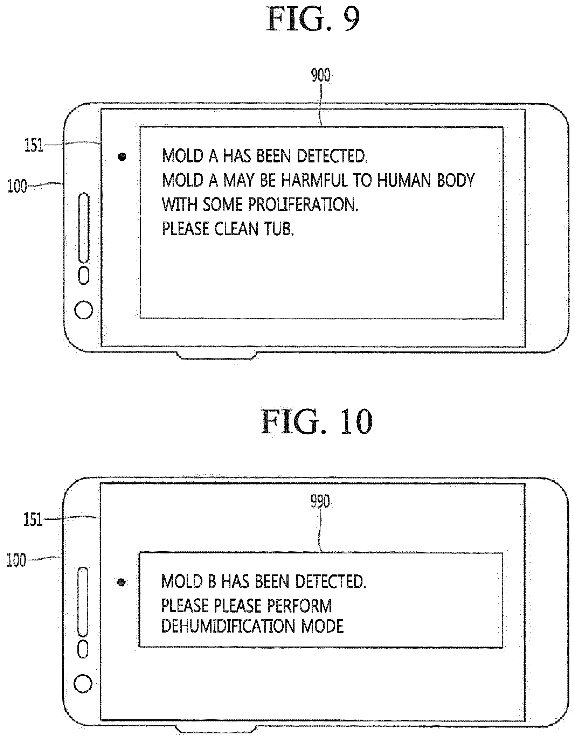

Referring to FIG. 9, the terminal 100 may display guide information 900 including microorganism information indicating <Mold A has been detected> and laundry guide information indicating <Please clean the tub> on the display 151.

Referring to FIG. 10, the terminal 100 may display guide information 1000 including microorganism information «Mold B has been detected> and laundry guide information indicating <Please perform dehumidification mode> on the display 151.

The user may check the bacterial condition of the laundry treatment device 300 through the guide information 900 and 990 and immediately take measures accordingly.

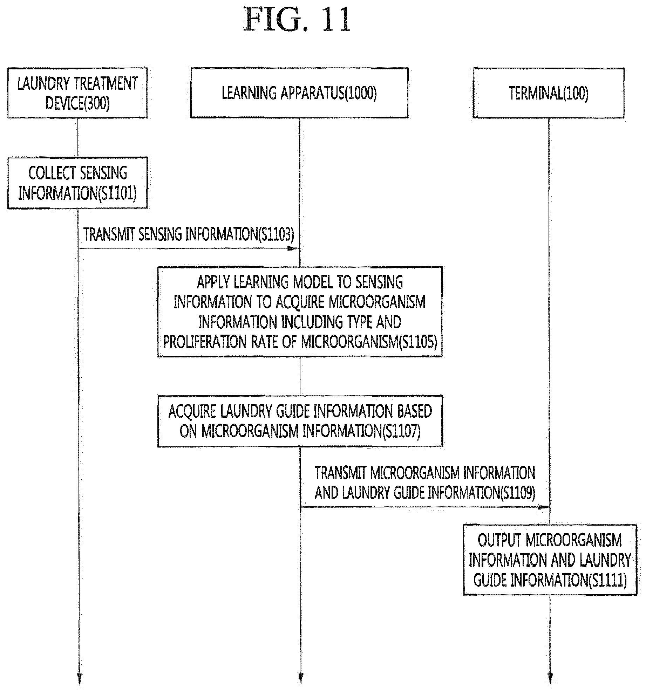

FIG. 11 is a ladder diagram illustrating a method of operating a washing system according to an embodiment of the present invention.

Particularly, FIG. 11 is a diagram illustrating an embodiment in which microorganism information and laundry guide are acquired by the learning apparatus 1000.

Referring to FIG. 11, the processor 180 of the laundry treatment device 300 collects sensing information through the sensing unit 140.

In one embodiment, the sensing unit 140 may include at least one of a mold sensor (not shown), an air quality sensor (not shown) or a gas sensor.

In addition, one or more mold sensors, one or more air quality sensors or one or more gas sensors may be provided inside the laundry treatment device 300.

The sensing information may be collected in order to determine the type of microorganism or the proliferation rate of the microorganism such as mold or bacteria.

The sensing information may include a sensing value measured through the mold sensor, a sensing value measured through the air quality sensor and information on a time when each sensing value is measured.

The processor 180 of the laundry treatment device 300 transmits the collected sensing information to the learning apparatus 1000 through the wireless communication unit 110 (S1103).

In one embodiment, the processor 180 may periodically transmit the sensing information to the learning apparatus 1000.

The learning apparatus 1000 applies a learning model to the received sensing information to acquire microorganism information including the type of the microorganism and the proliferation rate of the microorganism (S1105).

The learning apparatus 1000 may generate the learning model based on the collected sensing information.

The learning model may be a model in which relationships between sensing information and the types of microorganisms are learned using a linear regression algorithm.

In another embodiment, the learning model may be a model in which relationships between sensing information, the types of the microorganisms and the proliferation rates of the microorganism are learned using a linear regression algorithm.

The learning apparatus 1000 acquires laundry guide information based on the acquired microorganism information (S1107).

The learning apparatus 1000 may pre-store a table associating the laundry guide information with the type of the microorganism and the proliferation rate of the microorganism in a database.

The learning apparatus 1000 may acquire the laundry guide information corresponding to the type of the microorganism and the proliferation rate of the microorganism using the table stored in the database, when the type of the microorganism and the proliferation rate of the microorganism are determined.

The learning apparatus 1000 transmits the acquired microorganism information and the laundry guide information to the terminal 100 (S1109).

In another example, the learning apparatus 1000 may transmit the acquired microorganism information and the laundry guide information to the laundry treatment device 300 and the laundry treatment device 300 may transmit the microorganism information and the laundry guide information to the terminal 100.

The terminal 100 outputs the microorganism information and the laundry guide information received from the learning apparatus 1000 through the display 151 (S1111).

The user may confirm which microorganism is present in the laundry treatment device 300 and at which proliferation rate the number of microorganisms is increased, through the microorganism information.

In addition, the user may be guided through the laundry guide information to determine what action is taken against the laundry treatment device 300. The user may operate the laundry treatment device 300 according to the laundry guide information, thereby preventing the laundry treatment device 300 from being damaged and preventing laundry from being contaminated by microorganisms.

According to the embodiment of the present invention, washing operation suitable for the type and proliferation rate of microorganism detected in the laundry treatment device is performed, it is possible to more accurately perform washing operation.

Therefore, it is possible to reduce the degree of contamination of laundry and to increase the lifespan of the laundry treatment device.

The present invention may be implemented as code that can be written to a computer-readable recording medium and can thus be read by a computer. The computer-readable recording medium may be any type of recording device in which data can be stored in a computer-readable manner. Examples of the computer-readable recording medium include a hard disk drive (HDD), a solid state drive (SSD), a silicon disk drive (SDD), a ROM, a RAM, a CD-ROM, a magnetic tape, a floppy disk, optical data storage, and a carrier wave (e.g., data transmission over the Internet). In addition, the computer may include the first controller 180 of the terminal.

## Claims

1. A laundry treatment device (300) comprising:
a wireless communication interface;
at least one sensor; and
a processor (180),
**characterized in that** the processor (180) is configured to:
apply a learning model learned through a supervised learning algorithm to sensing information including a sensing value collected from the at least one sensor and a measurement time of the sensing value,
acquire microorganism information including a type of microorganism and a proliferation rate of the microorganism,
acquire laundry guide information based on the acquired microorganism information, and
transmit the acquired laundry guide information to a terminal through the wireless communication interface.

2. The laundry treatment device (300) of claim 1, wherein the learning model includes a relationship among sensing information, the type of microorganism and the proliferation rate of the microorganism.

3. The laundry treatment device (300) of claim 2, wherein the processor (180) applies the learning model to the sensing information collected through the at least one sensor as input data and acquires the type of the microorganism and the proliferation rate of the microorganism suiting the input data.

4. The laundry treatment device (300) of claim 3, further comprising a memory (170) configured to store a table associating a laundry guide with the type of the microorganism and the proliferation rate of the microorganism.

5. The laundry treatment device (300) of any one preceding claim, wherein the processor (180) acquires a laundry guide corresponding to the type of the microorganism and the proliferation rate of the microorganism using the table.

6. The laundry treatment device (300) of claim 1, wherein the laundry guide information includes information for guiding specific laundry operation in order to eliminate the microorganism or prevent proliferation of the microorganism.

7. The laundry treatment device (300) of claim 6, wherein the laundry guide information further includes information on a position where the microorganism is detected.

8. A method of operating a laundry treatment device (300), the method comprising:
collecting sensing information including a sensing value collected from at least one sensor and a measurement time of the sensing value;
applying a learning model learned through a supervised learning algorithm to the sensing information to acquire microorganism information including a types of microorganism and a proliferation rate of the microorganism; and
acquiring laundry guide information based on the acquired microorganism information and transmitting the acquired laundry guide information to a terminal.

9. The method of claim 8, wherein the learning model includes a relationship among sensing information, the type of microorganism and the proliferation rate of the microorganism.

10. The method of claim 9, further comprising applying the learning model to the sensing information collected through the at least one sensor as input data; and
acquiring the type of the microorganism and the proliferation rate of the microorganism suiting the input data.

11. The method of claim 10, further comprising storing a table associating a laundry guide with the type of the microorganism and the proliferation rate of the microorganism.

12. The method of claim 11, further comprising acquiring a laundry guide corresponding to the type of the microorganism and the proliferation rate of the microorganism using the table.

13. The method of any one of claims 8 to 12, wherein the laundry guide information includes information for guiding specific laundry operation in order to eliminate the microorganism or prevent proliferation of the microorganism.

14. The method of claim 13, wherein the laundry guide information further includes information on a position where the microorganism is detected.

15. A recording medium having recorded thereon a program for performing a method of operating a laundry treatment device, the method comprising:
collecting sensing information including a sensing value collected from at least one sensor and a measurement time of the sensing value;
applying a learning model learned through a supervised learning algorithm to the sensing information to acquire microorganism information including a types of microorganism and a proliferation rate of the microorganism; and
acquiring laundry guide information based on the acquired microorganism information and transmitting the acquired laundry guide information to a terminal.
